# EUROPEAN PATENT SPECIFICATION

(11) **EP 1 287 747 B1**
(45) Date of publication and mention of the grant of the patent: **08.06.2005**
(21) Application number: 02016833.2
(22) Date of filing: 29.07.2002
(51) Int. Cl.: A23G 9/00, A23G 9/22, A23G 9/02, A23L 1/0532, A61K 9/08

(54) **Quiescently frozen ice products**
Im Ruhezustand Gefrorenes
Produits congelés en condition non-mouvement

(30) Priority: 29.08.2001 US 315938 P
(43) Date of publication of application: 05.03.2003
(73) Proprietor: Warner-Lambert Company LLC, Morris Plains, New Jersey 07950 (US)
(72) Inventor: Somani, Jitendra Krishan, Toronto M1L 2N3, Ontario (CA); Levi, Luigi, Toronto M1L 2N3, Ontario (CA); Lee, Joseph, Toronto M1L 2N3, Ontario (CA); Ramsay, Michael Paul, Toronto M1L 2N3, Ontario (CA)
(74) Representative: Tesch, Rudolf

(56) References cited:
- WO-A-00/61110
- WO-A-02/094035
- US-A- 4 725 445
- US-A- 5 290 605
- US-A- 5 358 728
- US-A- 5 525 352
- SIRAKAWA M ET AL: "Food for helping intake/swallowing is produced by adding reversible at-res heat-responsive galactoxyloglucan to drink water or liquid foods, an heating" DERWENT, XP002236009

## Description

The present invention relates to quiescently frozen ice products and their production. More specifically, the present invention relates to frozen ice products containing salts or esters of alginic acid for improved freezability.

### BACKGROUND OF THE INVENTION

Frozen ice products have been apart of our culture for quite some time. From the "snowball" to the more familiar italian ices, freezer pops and frozen fruit juices, these frozen novelties have long been a popular item with consumers. Frozen products of this type are, typically, carried on sticks, in plastic tubes or in wax coated paper containers and eaten directly from the freezer. No utensils or plates are necessary. And, recently, these products have enjoyed even greater commercial success due in part to the public's heightened awareness of such health concern as calories, cholesterol, fat and chemical-additives.

The use of frozen ice products to deliver drug or nutritional agents has also gained in popularity over the last few years. Such products are disclosed in e.g., US 5 525 352; WO 00 61110; and US 5 358 728. Due to the nature of the ingredients involved, various safeguards are being considered which restrict child access to such products.

One attempt at providing such a safeguard considers the possibility of storing the active containing ice products in child resistant box or box-like containers or cartons. However, at solid contents greater than 8% of the ice product formulation and at temperatures above -20°C, such containers tend to inhibit the freezing of the ice product composition. Either the ice products fail to freeze properly or remain liquid. Hence, a need exists for frozen product formulations which freeze solidly and uniformly in package containers.

Present inventors have found that by incorporating salts or esters of alginic acid the freezability (i.e., ability to freeze) of frozen ice products in containers or cartons is improved such that such containers remain a viable option for restricting access to medicated frozen ice products.

Accordingly an aspect of the present invention is to provide improved frozen ice products.

Another aspect of the present invention is to provide frozen ice product formulations which facilitate freezing of the frozen ice product at temperatures of from -5°C to -20°.

Still another aspect of the present invention is to provide frozen ice product formulations of specific solids content comprising pharmaceutically acceptable salts of alginic acid to facilitate freezing of the frozen ice product at temperatures of from -5°C to - 20°.

These and other aspects of the present invention are described in detail below.

### SUMMARY OF THE INVENTION

The present invention in one of its aspects relates to frozen compositions, comprising:
a.) an amount of a freezing facilitator consisting essentially of a pharmaceutically acceptable salt or ester of alginic acid such that the viscosity of the composition prior to freezing is no more than 300 cps;
b.) a safe and effective amount of pharmaceutical active;
c.) optionally, a flavor;
d.) optionally, a sweetener; and
e.) water
wherein the frozen composition contains a solids content greater than 8% and less than 30%, preferably less than 25% (or about 25%) and most preferably a solids content of from 9% (or about 9%) to 20% (or about 20%) and wherein the frozen compositions are frozen quiescently within the container at a temperature range of from -5°C (or about -5°C) to about -20°C (or about -20°C).

All percentages and ratios used herein are by weight unless otherwise specified. Additionally, all measurements are made at 25°C. unless otherwise specified.

The compositions of the present invention can comprise, consist essentially of, or consist of, the essential as well as optional ingredients and components described herein. As used herein, "consisting essentially of" means that the composition or component may include additional ingredients, but only if the additional ingredients do not materially alter the basic and novel characteristics of the claimed compositions or methods.

The phrase "safe and effective amount", as used herein, is defined as an amount of a substance sufficient to provide the desired benefit without undue adverse side effects, such as toxicity, irritation, or allergic response, commensurate with a reasonable benefit/risk ratio when used in the manner of this invention.

The term "pharmaceutically acceptable salts", as used herein, means salts of alginic acid which have the same general properties as the compounds from which they are derived, and which are acceptable from a toxicity viewpoint.

The term "frozen," as used herein, means that the product is solidified under freezing conditions, with or without air incorporation to a harden, firm consistency.

The term "quiescently frozen" as used herein means that the product has been statically frozen, that is, frozen without the accompaniment of stirring or agitation.

### DETAILED DESCRIPTION OF THE INVENTION

The essential as well as optional components of the capsules of the present invention are described in the following paragraphs.

### Essential Components

### Salts of Alginic Acid

An essential component of the present invention is a pharmaceutically acceptable salt of alginic acid. Alginic acid is a polysaccharide made up of D-mannuronic acid and L-guluronic acid residues. It is produced from brown seaweed (Phaeophycae) through a variety of extraction processes. Depending on the particular species of seaweed and the method used in extraction, alginates of various molecular weights can be prepared.

It has now been found that pharmaceutically acceptable salts of alginic acid, particularly water-soluble salts of alginic acid facilitate the freezing of frozen ice products. The water-soluble alginates used in the present invention are generally the alkali metal salts of alginic acid such as potassium or sodium alginate. Also useful herein are esters of alginic acid such as propylene glycol esters of alginic acid (e.g., 1,2-propylene glycol esters). Preferred for use herein is sodium alginate. Useful grades of alginic acid include the Manugel series of alginates such as Manugel DMB, supplied by ISP Technologies, Proctin series supplied by FMC Biopolymer, the Protanal series of alginates (available with varying specifications of viscosity) such as Protanal LF 5/60, Protanal LF 10/60 and Protanal LF200 (M, DL or S), supplied by FMC Biopolymer as well as the Protanal Ester series supplied by FMC Biopolymer and the Kelcoloid series of alginate esters such as Kelcoloid LVF (propane-1,2-diol alginate) supplied by ISP Technologies (ISP Alginates). Preferred for use herein are the Protanal LF200 grades M, DL and S.

The esters and/or salts of alginic acid are incorporated into the frozen compositions of the present invention at amounts such that the viscosity of the frozen compositions is no more than 300 cps, preferably from 25 cps (or about 25 cps) to 200 cps (or about 200 cps), more preferably from 50 cps (or about 50 cps) to 150 cps (or about 150 cps) and most preferably from 75 cps (or about 75 cps) to 125 cps (or about 125 cps) as measured at 25°C using a Brookfield viscometer Model LVT with a #1 spindle at 30 rpm.

It should be understood that different grades of salts or esters of alginic acid as well as mixtures thereof can be incorporated at varying amounts by persons skilled in the art to achieve the above-mentioned viscosities.

### Water

Water is also present in the frozen ice products of the present invention. Water comprises from about 50% to about 95%, preferably from about 70% to about 90% of the frozen ice products described herein. These amounts of water include the free water which is added, plus that amount which is introduced with other materials such as with corn syrup. The water, used in the present invention should preferably be deionized, distilled, free of organic impurities and bacteria and substantially free of metal ions.

The frozen ice products of the present invention includes one or more pharmaceutically active ingredients. The active ingredient may be at least one member selected from the group consisting of nourishing and health-promoting agents, antipyretic-analgesic-inflammatory agents (such as aspirin, NSAIDS and acetaminophen), antipsychotic drugs, antianxiety drugs, antidepressants, hypnotic-sedative agents, spasmolytics, gastrointestinal function conditioning agents, antacids, antitussive-expectorants (such as dextromethorphan and guaifenesin), dental buccal drugs, antihistamines, cardiotonics, antiarrhythmic drugs, diuretics, antihypertensive drugs, vasoconstrictors, coronary vasodilators, peripheral vasodilators, cholagogues, antibiotics, chemotherapeutic drugs, antidiabetic agents, drugs for osteoporosis, skeletal muscle relaxants and mixtures thereof. A more detailed list of pharmaceutical actives suitable for use in the present invention can be found in U.S. Patent 5,720,974, issued February 24, 1998.

Demulcents can also be used in the frozen compositions of the present invention. Demulcents are usually water-soluble polymers which are applied topically to protect, lubricate and coat the mucous membrane surfaces of the throat to relieve irritation. Suitable demulcents include demulcents such as elm bark, glycerin, pectin and mixtures thereof.

A food grade acid is generally employed in the frozen ice products of the present invention to improve taste. The food grade acid may be chosen from, but not limited to, the group consisting of adipic, fumaric, citric, tartaric or any other food grade acid known to the art. A combination of food acids from the above group is suitable for the invention. Generally, food grade acid is added at a level of from 0.2% to 0.7% by weight of the frozen confection, and typically from 0.3% to 0.6% by weight. A buffer, such as trisodium citrate, disodium phosphate, potassium phosphate, sodium tartrate, etc., may additionally be included for pH process control purposes. The pH of the confection generally ranges from 3.0-5.0 and is not considered critical to the functionality of the invention, but rather is important organoleptically.

A sweetener may also be incorporated into the frozen ice products of the present invention. Carbohydrate sweeteners, such as sucrose, dextrose, fructose, glucose, etc., are most often employed. Carbohydrate sweeteners are generally included at a level of 5 to 50% by weight of the frozen confection, and preferably 5 to 20% by weight. Carbohydrate sweeteners so employed additionally act to depress the freezing point of the frozen confection such that the frozen confection is ready-to-eat at freezer temperatures. Optionally, freezing point depressants such as salts or polyhydric alcohols may be employed to further depress the frozen confection freezing point. However, it is preferred that said carbohydrate sweeteners not be supplemented with specific freezing point depressants.

In a preferred embodiment of the invention, a non-nutritive sweetener such as saccharin, cyclamate, acetosulfame, aspartame, sucralose or a combination thereof, may be used in place of or in addition to carbohydrate sweeteners. Generally, said non-nutritive sweeteners is added at a level of 0.03% to 0.15% by weight, and typically from 0.06% to 0.10% by weight of the frozen confection. A freezing point depressant is necessary in the absence of carbohydrate solids to depress the freezing point of the frozen confection. A freezing point depressant may be chosen from the group consisting of sodium chloride, glycerin, propylene glycol and polyhydric alcohols, and is generally employed at a level of 2% to 5% of the frozen confection.

Natural and/or artificial flavors (e.g. fruit flavors) may also be added to the hydrocolloid mix at levels as would be obvious to those skilled in the art. Also, natural and/or artificial colors may similarly be employed. Optionally, natural fruit juices such as strawberry, cherry, orange, grape, raspberry, blueberry, apple, watermelon, banana, pineapple, cranberry, blackberry, lemon, grapefruit, lime, coconut, pear, peach, etc. may be incorporated to the frozen confection at levels ranging from 0.1% to 15% and typically from 7% to 13% by weight of the final frozen confection.

Souring agents can also be added, depending upon the flavoring agent involved. Any souring agent can be used within the scope of the present invention. Citric acid is an example of a typical souring agent. Malic or Tartaric acid can also be used. The concentration of the souring agent depends again on the flavoring agent(s) and desired taste. Preferably 0 to 0.5% by weight souring agent is used.

The compositions of the present invention preferably contain food colorings. These food colorings include U.S. Certified Food Colors Red #40, Yellow #6, Blue #1. Although not required, food colorings are included to enhance the aesthetic desirability of the composition. Food coloring also helps consumers associate the color with the flavor. For example, raspberry flavor can be dyed red and grape flavor can be dyed blue. A full recitation of all FD&C and D&C colorants useful in the present invention and their corresponding chemical structures can be found in the Kirk-Othmer Encyclopedia of Chemical Technology, 3rd Edition, in Volume 6, at pages 561-595, herein incorporated by reference.

The frozen products of the present invention preferably do not form gels or gel-like networks. Consequently, the frozen compositions of the present invention are preferably substantially free of gelation catalysts such as potassium or calcium as well as such gel forming stabilizers as guar gum, xanthan gum, locust bean gum, gum aragic, carboxymethyl cellulose, methyl cellulose, hydroxy propyl cellulose, hydroxy propyl methyl cellulose, collagen or mixtures thereof. By "substantially free " is meant that the compositions comprise less than an amount of such gelling ingredients that will adversely affect the dissolution properties of the prevent invention. Generally, this will mean that the present compositions comprise no more than about 0.02%, preferably no more than about 0.01%, of such materials. Without being limited by theory, it is believed that gelation prolongs product dissolution time in the mouth which can, in turn, prolong undesirable mouthfeel and taste. The frozen compositions of the present invention are preferably fast dissolving. By "fast dissolving" is meant that the frozen compositions dissolve in less than about 30 seconds, preferably less than about 15 seconds, more preferably less than about 10 seconds, after placing the frozen composition in the oral cavity.

The above-described compositions are frozen. To freeze the above-described compositions, the compositions are placed in an environment at a temperature from -5°C to about -20°C. In a preferred embodiment, the compositions are frozen and/or kept frozen in a container selected from the group consisting of plastic bags, paper bags, cardboard boxes, waxed paper, foam container and plastic containers.

The_following examples further describe and demonstrate preferred embodiments of the present invention.

### EXAMPLES

The following compositions are representative of the present invention.

### Examples I-IV

Using conventional mixing technology, the following frozen ice product compositions of the present invention are formulated from the ingredients listed below poured into a suitable single serving or unit dose package, place in a carton or container with at least one other single serving packaged frozen ice product and, then frozen in a multi-package container or carton. Alternatively, the single serving packaged frozen ice product may be frozen prior to storage in the multi-package container or carton.

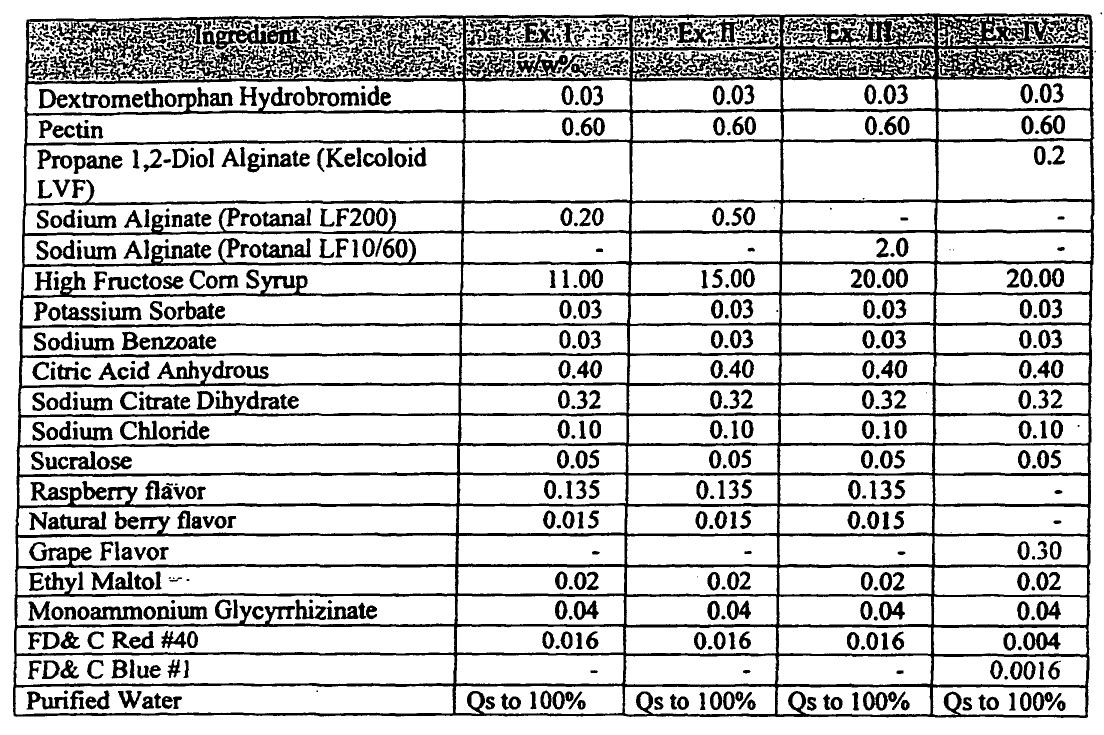

## Claims

1. A quiescently frozen ice composition, comprising:
a.) an amount of a freezing facilitator consisting essentially of a pharmaceutically acceptable salt or ester of alginic acid such that the viscosity of the composition prior to freezing is no more than 300cps; as measured at 25°C using a Brookfield viscometer Model LVT with a # 1 spindle at 30 rpm;
b.) one or more pharmaceutically active ingredient;
c.) optionally, a flavor;
d.) optionally, a sweetener; and
e.) water
wherein the frozen composition contains a solids content of greater than 8% and less than 30% and wherein the frozen compositions are frozen quiescently at a temperature range of from -5°C to about -20°C.

2. A frozen composition according to Claim 1, wherein the ester of alginic acid is a propylene glycol ester of alginic acid.

3. A frozen composition according to Claim 1 wherein the solids content is less than 25%.

4. A frozen composition according to Claim 1 wherein the solids content is from 9 to 20%.

5. A frozen composition according to Claim 1, wherein the viscosity of the composition is froin 25 cps to 200 cps.

6. A frozen composition according to Claim 5, wherein the viscosity of the composition is from 50 cps to 150 cps.

7. A frozen composition according to Claim 1 wherein the alginic acid salt is selected from the group consisting of potassium alginate, sodium alginate and mixtures thereof.

8. A frozen composition according to Claim 7 wherein the alginic acid salt is sodium alginate.

9. A frozen composition according to Claim I, wherein the pharmaceutically active ingredient is selected from the group consisting of antipyretic-analgesic-inflammatory agents, antipsychotic drugs, antianxiety drugs, antidepressants, hypnotic-sedative, agents, spasmolytics, gastrointestinal function conditioning agents, antacids, antitussive-expectorants, dental buccal drugs, antihistamines, cardiotonics, antiarrhythmic drugs, diuretics, antihypertensive drugs, vasoconstrictors, coronary vasodilators, peripheral vasodilators, cholagogues, antibiotics, chemotherapeutic drugs, antidiabetic agents, drugs for osteoporosis, skeletal muscle relaxants and mixtures thereof.

10. A frozen composition according to claim 1, further comprising a demulcent.

11. A container for storing quiescently frozen ice compositions, the container comprising quiescently frozen compositions, comprising:
a. an amount of a freezing facilitator consisting essentially of a pharmaceutically acceptable salt or ester of alginic acid such that the viscosity of the composition prior to freezing is no more than 300cps; as measured at 25°C using a Brookfield viscometer Model LVT with a # 1 spindle at 30 rpm;
b. one or more pharmaceutically active ingredient;
c. optionally, a flavor;
d. optionally, a sweetener; and
e. water
wherein the frozen composition contains a solids content of greater than 8% and less than 30% and wherein the frozen compositions are frozen quiescently and/or maintained in a frozen state at a temperature range of from -5°C to about -20°C

12. A container for storing frozen composition according to Claim 11, wherein the container is selected from the group consisting of plastic bags, paper bags, cardboard boxes, waxed paper, foam containers and plastic containers.

13. Use of a safe and effective amount of a composition according to claim 1 in the manufacture of a product for preventing or treating the symptoms of a gastrointestinal disorder by administering to an individual having difficultly swallowing tablets or capsules.

14. Use of a safe and effective amount of a composition according to claim 1 in the manufacture of a product for preventing or treating the symptoms of a respiratory illness by administering to an individual having difficultly swallowing tablets or capsules.

## Patentansprüche

1. Im Ruhezustand (quiescently) gefrorene Eiszusammensetzung, die umfasst:
a) eine derartige Menge eines Gefrieren ermöglichenden Mittels, das im Wesentlichen aus einem pharmazeutisch akzeptablen Salz oder Ester von Alginsäure besteht, derart dass die Viskosität der Zusammensetzung vor dem Gefrieren nicht mehr als 300 cps beträgt, was bei 25 °C unter Verwendung eines Brookfield-Viskosimeters Modell LVT mit einer Spindel Nr. 1 mit 30 rpm (Umdrehung pro Minute) ermittelt wird;
b) einen oder mehrere pharmazeutische Wirkstoffe;
c) optional einen Aromastoff;
d) optional ein Süßungsmittel und
e) Wasser,
wobei die gefrorene Zusammensetzung einen Feststoffgehalt von größer als 8 % und weniger als 30 % enthält und wobei die gefrorenen Zusammensetzungen im Ruhezustand in einem Temperaturbereich von -5 °C bis etwa -20 °C gefroren werden.

2. Gefrorene Zusammensetzung nach Anspruch 1, wobei der Alginsäureester ein Propylenglykolester von Alginsäure ist.

3. Gefrorene Zusammensetzung nach Anspruch 1, wobei der Feststoffgehalt weniger als 25 % beträgt.

4. Gefrorene Zusammensetzung nach Anspruch 1, wobei der Feststoffgehalt 9 bis 20 % beträgt.

5. Gefrorene Zusammensetzung nach Anspruch 1, wobei die Viskosität der Zusammensetzung 25 cps bis 200 cps beträgt.

6. Gefrorene Zusammensetzung nach Anspruch 5, wobei die Viskosität der Zusammensetzung 50 cP bis 150 cP beträgt.

7. Gefrorene Zusammensetzung nach Anspruch 1, wobei das Alginsäuresalz aus der aus Kaliumalginat, Natriumalginat und Gemischen derselben bestehenden Gruppe ausgewählt ist.

8. Gefrorene Zusammensetzung nach Anspruch 7, wobei das Alginsäuresalz Natriumalginat ist.

9. Gefrorene Zusammensetzung nach Anspruch 1, wobei der pharmazeutische Wirkstoff aus der Gruppe von Antipyretika/Analgetika/entzündungshemmenden Mitteln, Antipsychotika, Antiangstarzneimitteln, Antidepressiva, hypnotisch-sedativen Mitteln, Spasmolytika, die Gastrointestinalfunktion konditionierenden Mitteln, Antazida, Antitussiva/Expektorantien, bukkalen Zahnarzneimitteln, Antihistaminika, Kardiotonika, Antiarrhythmika, Diuretika, Antihypertonika, Vasokonstriktoren, Koronarvasodilatatoren, peripheren Vasodilatatoren, Cholagoga, Antibiotika, Chemotherapeutika, Antidiabetika, Arzneimitteln für Osteoporose, Skelettmuskelrelaxantien und Gemischen derselben ausgewählt ist.

10. Gefrorene Zusammensetzung nach Anspruch 1, die ferner ein Demulcens umfasst.

11. Behälter zur Aufbewahrung von im Ruhezustand gefrorenen Eiszusammensetzungen, wobei der Behälter im Ruhezustand gefrorene Zusammensetzungen umfasst, die umfassen:
a) eine derartige Menge eines Gefrieren ermöglichenden Mittels, das im Wesentlichen aus einem pharmazeutisch akzeptablen Salz oder Ester von Alginsäure besteht, derart dass die Viskosität der Zusammensetzung vor dem Gefrieren nicht mehr als 300 cps beträgt, was bei 25 °C unter Verwendung eines Brookfield-Viskosimeters Modell LVT mit einer Spindel Nr. 1 mit 30 rpm.(Umdrehung pro Minute) ermittelt wird;
b) einen oder mehrere pharmazeutische Wirkstoffe;
c) optional einen Aromastoff;
d) optional ein Süßungsmittel und
e) Wasser,
wobei die gefrorene Zusammensetzung einen Feststoffgehalt von größer als 8 % und weniger als 30 % enthält und wobei die gefrorenen Zusammensetzungen in einem Temperaturbereich von -5 °C bis etwa -20 °C im Ruhezustand eingefroren und/oder in einem gefrorenen Zustand gehalten werden.

12. Behälter zur Aufbewahrung einer gefrorenen Zusammensetzung nach Anspruch 11, wobei der Behälter aus der Gruppe von Kunststoffbeuteln, Papierbeuteln, Kartonschachteln, Wachspapier, Schaumstoffbehältern und Kunststoffbehältern ausgewählt ist.

13. Verwendung einer sicheren und wirksamen Menge einer Zusammensetzung nach Anspruch 1 bei der Herstellung eines Produkts zur Prävention oder Behandlung der Symptome einer gastrointestinalen Störung durch Verabreichen an ein Individuum, das Schwierigkeiten mit dem Schlucken von Tabletten oder Kapseln hat.

14. Verwendung einer sicheren und wirksamen Menge einer Zusammensetzung nach Anspruch 1 bei der Herstellung eines Produkts zur Prävention oder Behandlung der Symptome einer Atemwegserkrankung durch Verabreichen an ein Individuum, das Schwierigkeiten mit dem Schlucken von Tabletten oder Kapseln hat.

## Revendications

1. Composition de glace soumise à une congélation statique, comprenant
a.) une quantité d'un facilitateur de la congélation constitué essentiellement d'un sel ou d'un ester de l'acide alginique pharmaceutiquement acceptable, de telle sorte que la viscosité de la composition, avant la congélation, n'est pas supérieure à 300 cps, telle qu'on la mesure à 25 °C en utilisant un viscosimètre de Brookfield modèle LVT avec une broche # 1 à 30 tours/minute ;
b.) un ou plusieurs ingrédients pharmaceutiquement actifs ;
c.) à titre facultatif, un arôme ;
d.) à titre facultatif, un édulcorant ; et
e.) de l'eau
la composition congelée possédant une teneur en produits solides supérieure à 8 % et inférieure à 30 % et les compositions congelées étant soumises à une congélation statique dans la plage de températures de -5 °C à environ -20 °C.

2. Composition congelée selon la revendication 1, dans laquelle l'ester de l'acide alginique est un ester de propylèneglycol de l'acide alginique.

3. Composition congelée selon la revendication 1, dans laquelle la teneur en produits solides est inférieure à 25 %.

4. Composition congelée selon la revendication 1, dans laquelle la teneur en produits solides s'élève de 9 à 20 %.

5. Composition congelée selon la revendication 1, dans laquelle la viscosité de la composition s'élève de 25 cps à 200 cps.

6. Composition congelée selon la revendication 5, dans laquelle la viscosité de la composition s'élève de 50 cps à 150 cps.

7. Composition congelée selon la revendication 1, dans laquelle le sel de l'acide alginique est choisi parmi le groupe constitué par l'alginate de potassium, l'alginate de sodium et leurs mélanges.

8. Composition congelée selon la revendication 7, dans laquelle le sel de l'acide alginique est l'alginate de sodium.

9. Composition congelée selon la revendication 1, dans laquelle l'ingrédient pharmaceutiquement actif est choisi parmi le groupe constitué par des agents antipyrétiques-analgésiques-inflammatoires, des antipsychotiques, des anxiolytiques, des antidépresseurs, des nooleptiques, des spasmolytiques, des agents de conditionnement des fonctions gastro-intestinales, des antiacides, des antitussifs-expectorants, des médicaments bucco-dentaires, des antihistaminiques, des cardiotoniques, des médicaments antiarythmiques, des diurétiques, des antihypertenseurs, des vasoconstricteurs, des vasodilatateurs des coronaires, des vasodilatateurs périphériques, des cholagogues, des antibiotiques, des médicaments chimiothérapeutiques, des agents antidiabétiques, des médicaments contre l'ostéoporose, des relaxants des muscles squelettiques et leurs mélanges.

10. Composition congelée selon la revendication 1, comprenant en outre un adoucissant.

11. Récipient pour le stockage de compositions de glaces soumises à une congélation statique, le récipient comprenant des compositions soumises à une congélation statique, comprenant :
a.) une quantité d'un facilitateur de la congélation constitué essentiellement d'un sel ou d'un ester pharmaceutiquement acceptable de l'acide alginique, de telle sorte que la viscosité de la composition, avant la congélation, n'est pas supérieure à 300 cps, telle qu'on la mesure à 25 °C en utilisant un viscosimètre de Brookfield modèle LVT avec une broche # 1 à 30 tours/minute ;
b.) un ou plusieurs ingrédients pharmaceutiquement actifs ;
c.) à titre facultatif, un arôme ;
d.) à titre facultatif, un édulcorant ; et
e.) de l'eau
la composition congelée possédant une teneur en produits solides supérieure à 8 % et inférieure à 30 % et les compositions congelées étant soumises à une congélation statique et/ou étant maintenues à l'état congelé dans la plage de températures de -5 °C à environ -20 °C.

12. Récipient pour stocker une composition congelée selon la revendication 11, dans lequel le récipient est choisi parmi le groupe constitué par des sacs en matière plastique, des sacs en papier, des boîtes en carton, du papier ciré, des récipients en mousse et des récipients en matière plastique.

13. Utilisation d'une quantité sûre et efficace d'une composition selon la revendication 1 dans la fabrication d'un produit pour prévenir ou pour traiter les symptômes d'un trouble gastro-intestinal par administration à un individu éprouvant des difficultés à avaler des comprimés ou des capsules.

14. Utilisation d'une quantité sûre et efficace d'une composition selon la revendication 1 dans la fabrication d'un produit pour prévenir ou pour traiter les symptômes d'une insuffisance respiratoire par administration à un individu éprouvant des difficultés à avaler des comprimés ou des capsules.
